(19) Europäisches Patentamt
European Patent Office
Office européen des brevets

(11) Veröffentlichungsnummer: **0 038 936**
**B1**

(12) # EUROPÄISCHE PATENTSCHRIFT

(45) Veröffentlichungstag der Patentschrift: **27.04.83**

(51) Int. Cl.³: **C 07 C 121/80,** A 61 K 31/275

(21) Anmeldenummer: **81102294.6**

(22) Anmeldetag: **26.03.81**

(54) **Neue 1-Aryloxy-3-alkylamino-2-propanole und Verfahren zu ihrer Herstellung.**

(30) Priorität: **25.04.80 DE 3015991**

(43) Veröffentlichungstag der Anmeldung:
**04.11.81 Patentblatt 81/44**

(45) Bekanntmachung des Hinweises auf die Patenterteilung:
**27.04.83 Patentblatt 83/17**

(84) Benannte Vertragsstaaten:
**AT BE CH DE FR IT LI LU NL SE**

(56) Entgegenhaltungen:
**CH-A- 516 509**
**DE-A-2 503 222**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Stähle, Helmut, Dr., Rotweinstrasse 23, D-6507 Ingelheim/Rhein (DE)**
Erfinder: **Muacevic, Gojko, Dr., In der Dörrwiese 13, D-6507 Ingelheim/Rhein (DE)**
Erfinder: **Traunecker, Werner, Dr., Birkenweg 1, D-6531 Münster-Sarmsheim (DE)**

**EP 0 038 936 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue 1-Aryloxy-3-alkylamino-2-propanole und Verfahren zu ihrer Herstellung

Die Erfindung betrifft neue Verbindungen der Formel

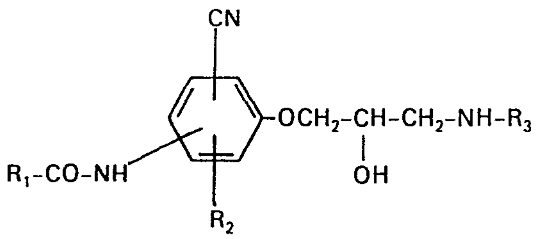

I

in der

$R_1$ einen geraden oder verzweigten Alkylrest mit 1 bis 20 C-Atomen

$R_2$ ein Wasserstoff- oder Halogenatom, eine gerade oder verzweigte Alkyl- oder Alkoxygruppe mit 1 bis 4 C-Atomen oder die zweiwertigen Gruppen $-CH=CH-CH=CH-$ oder $-(CH_2)_n-$ (n = ganze Zahl von 3 bis 5) mit Bindung der freien Valenzen in o-Stellung zueinander

$R_3$ einen geraden oder verzweigten Alkylrest mit 3 bis 10 C-Atomen, wobei $R_3$ von Isopropyl und von tert. Butyl verschieden ist, wenn $R_1$ Äthyl oder n-Propyl und gleichzeitig $R_2$ Wasserstoff bedeutet

ihre Säureadditionssalze, die Verwendung dieser Verbindungen in Arzneimitteln sowie ihre Herstellung.

Bevorzugte Bedeutungen für $R_1$ sind gerade oder verzweigte Alkylreste mit 1 bis 7 C-Atomen, insbesondere der n-Pentylrest.

Bevorzugte Bedeutung für $R_2$ ist Wasserstoff.

Bevorzugte Bedeutung für $R_3$ ist ein in der Nachbarschaft zur Aminogruppe verzweigter Alkylrest, insbesondere der Isopropyl- oder tert. Butylrest.

Die neuen Verbindungen können auf folgende Weise hergestellt werden:

a) Umsetzung einer Verbindung der allgemeinen Formel II

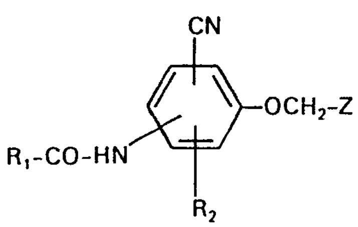

II

in der $R_1$ und $R_2$ wie in der Formel I definiert sind und Z die Gruppe

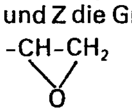

oder $-CHOH-CH_2-Hal$ (Hal = Halogen) bedeutet, mit einem Amin der allgemeinen Formel

$NH_2R_3$ III

in der $R_3$ die in Formel I angegebene Bedeutung hat.

b) Hydrolyse eines Oxazolidin-Derivates der allgemeinen Formel

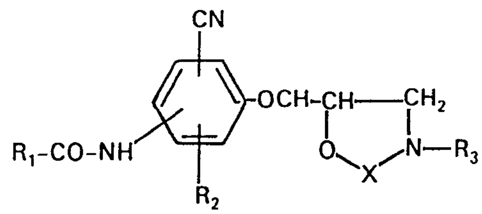

IV

in der $R_1$ bis $R_3$ wie in Formel I definiert sind und X eine $-CO-$, $-CH_2-$ oder -CH-niederalkyl-Gruppe bedeutet, beispielsweise mit Natron- oder Kalilauge in Wasser oder in einer Alkohol-Wasser-Mischung.

c) Umsetzung einer Verbindung der allgemeinen Formel V

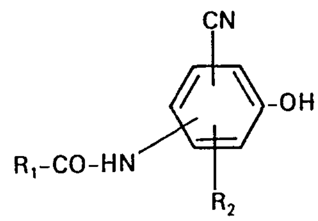

V

in welcher $R_1$ und $R_2$ die obengenannte Bedeutung haben, bzw. eines Salzes dieses Phenols, mit einem Azetidinolderivat der allgemeinen Formel VI

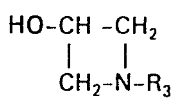

VI

in welcher $R_3$ die obengenannte Bedeutung hat, in wasserfreiem Medium.

Die Oxazolidinone der Formel IV (d.h. Verbindungen, bei denen Y = -CO- darstellt) sind beispielsweise ausgehend von den Epoxiden der Formel II herstellbar, indem man letztere mit einem (aus Chlorameisensäureäthylester und einem Amin der Formel III darstellbaren) Urethan der Formel VII

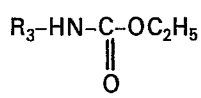

VII

in der $R_3$ die obenbezeichnete Bedeutung hat, umsetzt.

Die Ausgangsphenole der allgemeinen Formel V und die Azetidinole der allgemeinen Formel VI können nach literaturbekannten Methoden [letztere s. beispielsweise Chem. pharm. Bull. (Japan), Vol. 22 (7), 1974, Seite 1490] hergestellt werden.

Die erfindungsgemässen Verbindungen besitzen ein asymmetrisches C-Atom an der CHOH-Gruppe und kommen daher als Racemat wie

auch in Form der optischen Antipoden vor. Letztere können ausser durch Racematentrennung mit üblichen Hilfssäuren wie Dibenzoyl- (bzw. Dip-Toluyl-) D-Weinsäure oder D-3-Bromcampher-8-sulfonsäure auch durch Einsetzen von optisch aktivem Ausgangsmaterial erhalten werden.

Die erfindungsgemässen 1-Phenoxy-2-hydroxy-3-alkylaminopropane der allgemeinen Formel I können in üblicher Weise in ihre physiologisch verträglichen Säureadditionssalze überführt werden. Geeignete Säuren sind beispielsweise Salzsäure, Bromwasserstoffsäure, Schwefelsäure, Methansulfonsäure, Maleinsäure, Essigsäure, Oxalsäure, Milchsäure, Weinsäure oder 8-Chlortheophyllin.

Die Verbindungen der allgemeinen Formel I bzw. deren physiologisch verträgliche Säureadditionssalze haben im Tierversuch wertvolle therapeutische, insbesondere β-adrenolytische Eigenschaften gezeigt und können daher beispielsweise zur Behandlung oder Prophylaxe koronarer Herzkrankheiten, insbesondere der Angina pectoris und zur Behandlung von Herzarrhythmien, insbesondere von Tachycardien, in der Humanmedizin eingesetzt werden.

Auch die blutdrucksenkenden Eigenschaften der Verbindungen sind therapeutisch interessant, zudem entfalten die Verbindungen der Formel I günstige metabolische Eigenschaften. Die Verbindungen haben gegenüber bekannten β-Rezeptorenblockern, z.B. dem ähnlich strukturierten Handelsprodukt 1-(2-Acetyl-4-butyroylaminophenoxy) -2-hydroxy -3-isopropyl- aminopropan (Acebutolol) den Vorteil beträchtlich verminderter Toxizität, besserer Wirkung und hervorragender Organselektivität.

Die Messung dieser Parameter erfolgt dabei nach den folgenden Vorschriften:

1. Hemmung der Isoprenalintachycardie (aludrinantagonistische Wirkung)

Methode: Hemmung der tachycarden Reaktion auf eine Standard-Dosis Isoprenalin und Einfluss auf die basale Herzfrequenz durch steigende i.v. Dosen eines β-Adrenolytikums.

Tiermaterial: Meerschweinchen beiderlei Geschlechts mit Körpergewichten von 270–350 g, Gruppenhaltung, Standard-Kost und Wasser bis zum Versuchsbeginn ad libitum. 16 Stunden vor dem Versuchsanfang Futterentzug.

Narkose: Äthylurethan 1,75 g/kg als 20%ige Lösung intraperitoneal, gegebenenfalls wurde nachinjiziert.

Präparation: Kanülierung einer Vena jugularis exterior für intravenöse Injektionen: Einbinden einer Trachealkanüle und künstliche Beatmung; subcutane Nadelelektroden zur Aufnahme des EKG, in der Regel Extremitätenableitung II, Registriergeschwindigkeit 25 mm/sec; Rektalthermometer zur Kontrolle der Körpertemperatur, die mit einer Wärmelampe (Infrarotstrahler) auf 34 bis 36°C mit Hilfe einer elektronischen Automatikeinrichtung konstant gehalten wird.

Versuchsablauf: Die Herzfrequenz wird durch Auszählen der R-Zacken im EKG bestimmt, jeweils aus einer 3 bis 4 sec langen Registrierzeit. Etwa 30 min nach der Präparation wird im Abstand von 2 min 5mal die normale Herzfrequenz gemessen und gemittelt. Anschliessend wird als adrenerges Stimulans 1 µg/kg Isoprenalin i.v. injiziert und danach 3 min lang alle 30 sec die Herzfrequenz erneut registriert. Die Isoprenalininjektionen werden während des ganzen Versuches im Abstand von 30 min wiederholt. Bleibt die Spontanfrequenz etwa konstant und ist die tachycarde Reaktion auf die ersten 2 bis 3 Isoprenalin-Gaben gleichmässig, dann wird 15 min nach der letzten und 15 min vor der nächsten Isoprenalin-Reaktion die erste Dosis der Prüfsubstanz i.v. injiziert. Weitere in geometrischer Reihe ansteigende Dosen der Prüfsubstanz folgen in Abständen von 60 min bis eine markante Hemmung der Isoprenalin-Tachycardie erreicht ist.

2. Prüfung auf Cardioselektivität am wachen Meerschweinchen

Prinzip: Nach der Methode von D. Dunlop und R.G. Shanks [brit. J. Pharmacol. 32, 201 (1968)] werden wache Meerschweinchen einer tödlichen Dosis eines Histaminaerosols ausgesetzt. Durch Vorbehandlung mit Isoprenalin werden die Tiere vor der letalen Wirkung des Histamins geschützt. Ein β-Adrenolytikum hebt die Isoprenalinwirkung auf, so dass der Schutz vor dem Histaminbronchospasmus verlorengeht, falls es sich um eine nicht cardioselektive Substanz handelt. Zeigt eine am Herzen wirksame β-adrenolytische Substanz in diesem Test keinen Antagonismus gegen Isoprenalin, dann kann Cardioselektivität (für sog. $\beta_1$-Rezeptoren) angenommen werden.

Tiermaterial: Meerschweinchen beiderlei Geschlechts (6 Tiere pro Dosis), mit 350–400 g Körpergewicht, Gruppenhaltung. Standard-Futter und Wasser bis zum Versuchsbeginn ad libitum. 16 Stunden vor dem Versuchsanfang Futterentzug.

Versuchsablauf: Gruppen von je 6 Tieren (3 männliche + 3 weibliche) werden s.c. mit 5 oder mehr verschiedenen Dosen des β-Adrenolytikums behandelt. Fünfzehn Minuten später erhalten sie 0,1 mg/kg Isoprenalin contralateral s.c. injiziert. Nach weiteren 15 Minuten werden die Tiere in die zylindrische Kammer von 2 Liter Inhalt gesetzt, 45 Sekunden lang einem wässrigen Histaminaerosol (1,25%ig) ausgesetzt und anschliessend wird die Mortalität ausgewertet.

Auswertung: Die Letalität wird gegen den Logarithmus der Dosis aufgetragen und die $LD_{50}$ nach J. Litchfield und F. Wilcoxon (J. Pharmacol. Exp. Terap. 96, 99–113, 1949) ermittelt. Mit der $LD_{50}$ aus diesem Versuch und der cardialen $ED_{50}$ aus dem Versuch der Hemmung der Isoprenalintachycardie (narkot. Meerschweinchen) wird ein Selektivitätsquotient

$$\left(\frac{LD\,50}{ED\,50}\right)$$

gebildet. Eine Substanz wird als cardioselektiv angesehen, wenn der Quotient grösser als 1 ist.

Als wertvoll haben sich dabei insbesondere

solche Verbindungen der allgemeinen Formel I herausgestellt, bei denen $R_3$ einen Isopropyl- oder einen tertiär-Butylaminorest bedeutet (substituierte p-Acylamino-1-Phenoxy-3-alkyl-amino-2-propanole). Besonders wertvoll sind insbesondere das 1-(2-Cyano-4n-hexanoyl-amino)-phenoxy-3-tertiärbutyl-amino-2-propanol und das 1-(2-Cyano-4-n-hexanoylamino) -phenoxy-3-isopropylamino-2-propanol und seine Salze.

Die Einzeldosis der erfindungsgemässen Substanzen liegt bei 1 bis 300 mg, vorzugsweise 5 bis 100 mg (oral) bzw. 1 bis 20 mg (parenteral).

Die erfindungsgemässen Wirkstoffe können in die üblichen galenischen Anwendungsformen, wie Tabletten, Dragées, Lösungen, Emulsionen, Pulver, Kapseln oder Depotformen gebracht werden, wobei zu deren Herstellung die üblichen pharmazeutischen Hilfsstoffe sowie die üblichen Fertigungsmethoden herangezogen werden können. Entsprechende Tabletten können beispielsweise durch Mischen der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung eines Depoteffekts, wie Carboxypolymethylen, Carboxymethylcellulose, Celluloseacetatphthalat,oder Polyvinylacetat erhalten werden.

Die Tabletten können auch aus mehreren Schichten bestehen. Entsprechend können Dragées durch Überziehen von analog den Tabletten hergestellten Kernen mit üblicherweise in Drageéüberzügen verwendeten Mitteln, beispielsweise Kollidon oder Schellack, Gummi arabicum, Talk, Titandioxid oder Zucker, hergestellt werden. Zur Erzielung eines Depoteffekts oder zur Vermeidung von Inkompatibilitäten kann der Kern auch aus mehreren Schichten bestehen. Desgleichen kann auch die Dragéehülle zur Erzielung eines Depoteffekts aus mehreren Schichten bestehen, wobei die oben bei den Tabletten erwähnten Hilfsstoffe verwendet werden können.

Säfte der erfindungsgemässen Wirkstoffe bzw. Wirkstoffkombinationen können zusätzlich noch ein Süssungsmittel, wie Saccharin, Cyclamat, Glycerin oder Zucker, sowie ein geschmackverbesserndes Mittel, z.B. Aromastoffe, wie Vanillin oder Orangenextrakt, enthalten. Sie können ausserdem Suspendierhilfsstoffe oder Dickungsmittel, wie Natriumcarboxymethyl-cellulose, Netzmittel, beispielsweise Kondensationsprodukte von Fettalkoholen mit Äthylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Komplexonen, hergestellt und in Injektionsflaschen oder Ampullen abgefüllt.

Die Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen der dafür vorgesehenen Wirkstoffe bzw. Wirkstoffkombinationen mit üblichen Trägermitteln, wie Neutralfetten oder Polyäthylenglykol bzw. dessen Derivaten, herstellen.

Die erfindungsgemässen Verbindungen sind auch für die Kombination mit anderen pharmakodynamisch wirksamen Stoffen wie z.B. Coronardilatatoren, Sympathicomimetica, Herzglykosiden oder Tranquilizern geeignet.

Die folgenden Beispiele erläutern die Erfindung, ohne sie zu beschränken:

A. Herstellungsbeispiele

Beispiel 1

1-(2-Cyano-4-n-hexanoylamino-phenoxy)-3-isopropyl-amino-2-propanol

10 g (0,035 Mol) 1-(2-Cyano-4-hexanoylamino-phenoxy)-2,3-epoxipropan werden in 100 ml Äthanol gelöst und nach Zugabe von 8,3 g (0,14 Mol) Isopropylamin eine Stunde unter Rückfluss zum Sieden erhitzt. Nach Abdampfen des Lösungsmittels i.V. erstarrt der verbleibende Rückstand. Es wird zweimal aus Acetonitril umkristallisiert. Die weisse kristalline Base wird abgesaugt und getrocknet.
Ausbeute: 3,8 g, Fp: 115–117°C.

Beispiel 2

1-(2-Cyano-4-n-acetylamino-phenoxy)-3-tert.-butylamino-2-propanol

11 g 1-(2-Cyano-4-acetylamino-phenoxy)-2,3-epoxipropan werden zusammen mit 10 ml tert. Butylamin in 80 ml Äthanol 1,5 Stunden unter Rückfluss zum Sieden erhitzt. Nach Abdestillieren des Lösungsmittels i.V. wird der verbleibende Rückstand mit $H_2O$ digeriert, mit HCl angesäuert und Neutralstoffe mit Essigester extrahiert. Die wässrige Phase wird mit NaOH alkalisch gestellt und das anfallende Amin in Essigester aufgenommen. Nach Waschen der organischen Phase mit $H_2O$ und Trocknen über $MgSO_4$ wird das Lösungsmittel i.V. abdestilliert. Der Rückstand wird aus Acetonitril umkristallisiert.
Ausbeute: 5,9 g. Fp: 122–123°C, DC: einheitlich.

Beispiel 3

1-(2-Cyano-4-n-hexanoylamino-phenoxy)-3-tert.-butylamino-2-propanol-hydrochlorid

10 g (0,03 Mol) 1-(2-Cyano-4-hexanoylamino-phenoxy)-2,3-epoxipropan werden nach Lösen in 100 ml Äthanol mit 7,3 g (0,1 Mol) tert. Butylamin versetzt und eine Stunde unter Rückfluss zum Sieden erhitzt. Nach Abkühlen wird das Lösungsmittel i.V. abdestilliert und der Rückstand über eine Kieselgelsäule gereinigt (Lösungsmittelgemisch: Essigester/Isopropanol/$NH_4OH$ (14:6:1). Die einheitlichen Fraktionen werden vereinigt, mit verd. NaOH gewaschen, getrocknet und das Trockenmittel abgesaugt. Nach Verdampfen des Lösungsmittelgemisches wird der verbleibende Rückstand in Äther gelöst, mit alkoholischer HCl angesäuert und das ausfallende Kristallisat abgesaugt. Es wird aus Acetonitril unter Zugabe von Äther umkristallisiert.

Ausbeute: 4,6 g.
Das reinweisse Hydrochlorid schmilzt bei 143–145°C.

In Analogie zu Beispiel 3 wurden die als Beispiele 4 und 5 aufgeführten Verbindungen synthetisiert.

Beispiel 4

1-(2-Cyano-4-n-octanoylamino-phenoxy)-3-tert.butylamino-2-propanol-hydrochlorid

Aus 10 g (0,03 Mol) 1-(2-Cyano-4-octanoylamino-phenoxy-2,3-epoxipropan und 7,3 g (0,1 Mol) tert. Butylamin erhält man 2,8 g saubere Base und daraus das o.a. Hydrochlorid.
Fp: 155–157°C.

Beispiel 5

1-[2-Cyano-4-(3,3-dimethylbutyroyl)-amino-phenoxy]-3-isobutylamin-2-propanol-hydrochlorid

Nach Aminolyse von 7,5 g (0,026 Mol) 1-[2-Cyano-4-(3,3-dimethylbutyroyl)-phenoxy]-2,3-epoxipropan mit 7,3 g (0,1 Mol) Isobutylamin, Aufarbeitung und Fällung als Hydrochlorid werden 1,6 g des Salzes gewonnen.
Fp: 163–166°C.

Beispiel 6

1-[2-Cyano-4-(2-äthylhexanoyl)-amino-phenoxy]-3-isopropylamino-2-propanol-hydrochlorid

15 g (0,043 Mol) 1-[2-Cyano-4-(2-äthyl-hexanoyl)aminophenoxy]-3-chlor-2-propanol werden nach Lösen in 80 ml Äthanol und Zugabe von 14,4 ml (0,17 Mol) Isopropylamin zwei Stunden unter Rückfluss gekocht. Dann wird das Lösungsmittel abdestilliert und der Rückstand über eine Kieselgelsäule (Fliessmittelgemisch: Essigester/Isopropanol/$NH_4OH$, 14:6:1) gereinigt. Die erhaltene reine Base wird aus Essigester unter Zugabe von Petroläther umkristallisiert. Das Kristallisat wird in Acetonitril gelöst, mit alkoholischer Salzsäure angesäuert und durch Zugabe von Äther die Kristallisation eingeleitet. Das Kristallisat ist reinweiss.
Ausbeute: 2,9 g. Fp: 169–170°C.

In Analogie zu Beispiel 6 wurden die in den Beispielen 7 bis 15 genannten Verbindungen synthetisiert.

Beispiel 7

1-(2-Cyano-6-n-hexanoylamino-phenoxy)-3-isopropylamino-2-propanol-hydrochlorid

Aus 12,9 g (0,04 Mol) 1-(2-Cyano-6-hexanoylamino-phenoxy)-3-chlor-w-propanol und 13,6 ml (0,16 Mol) Isopropylamin werden 5,3 g Aminoalkohol erhalten, der durch Zugabe von HCl in das Hydrochlorid verwandelt wird.
Ausbeute: 4,7 g. Fp: 107°C.

Beispiel 8

1-(2-Cyano-6-n-pentanoylamino-phenoxy)-3-isopropylamino-2-propanol-hydrochlorid

Aus 11 g (0,036 Mol) des Chlorhydrins und 9,4 ml (0,11 Mol) Isopropylamin werden nach Zugabe von HCl 2,9 g Hydrochlorid gewonnen.
Fp: 105–107°C.

Beispiel 9

1-(2-Cyano-6-n-pentanoylamino-phenoxy)-3-tert.-butylamino-2-propanol-hydrochlorid

Aus 11 g (0,036 Mol) des Chlorhydrins und 11,6 ml (0,11 Mol) tert. Butylamin werden nach Zugabe von HCl 2,6 g Hydrochlorid erhalten.
Fp: 123–124°C.

Beispiel 10

1-[2-Cyano-4-(2-äthylhexanoyl)-aminophenoxy]-3-tert.butylamino-2-propanol-hydrochlorid

Aus 15 g (0,043 Mol) des Chlorhydrins und 18 ml (0,17 Mol) tert. Butylamin werden nach Zugabe von HCl zum Aminoalkohol 4,9 g Hydrochlorid erhalten.
Fp: 179–181°C.

Beispiel 11

1-[2-Cyano-4-(2,2-dimethylpropionyl)-amino-phenoxy]-3-tert.-butylamino-2-propanol-hydrochlorid

Aus 10,5 g (0,032 Mol) Chlorhydrin und 8,7 g (0,12 Mol) tert. Butylamin werden nach Zugabe von HCl 6,8 g Hydrochlorid gewonnen.
Fp: 225–228°C.

Beispiel 12

1-[2-Cyano-4-(2,2-dimethylpropionyl)-amino-phenoxy]-3-isopropylamino-2-propanol-hydrochlorid

Aus 10,5 g (0,032 Mol) Chlorhydrin und 13 ml (0,15 Mol) Isopropylamin werden nach Zugabe von HCl zum Aminoalkohol 5,7 g Hydrochlorid erhalten.
Fp: 177–180°C.

Beispiel 13

1-(2-Cyano-6-isobutyroylamino-phenoxy)-3-isopropylamino-2-propanol-oxalat

Aus 14,7 g (0,05 Mol) Chlorhydrin und 8,5 ml (0,1 Mol) Isopropylamin werden nach Zugabe von Oxalsäure (in Acetonitril gelöst) zum Aminoalkohol 3,7 g Oxalat erhalten.
Fp: 196–198°C.

Beispiel 14

1-(2-Cyano-6-isobutyroylamino-phenoxy)-3-tert.-butylamino-2-propanol-oxalat

Aus 14,7 g (0,05 Mol) Chlorhydrin und 10,5 ml (0,1 Mol) tert. Butylamin werden nach Zugabe von Oxalsäure zum Aminoalkohol 4,7 g Oxalat gewonnen.
Fp: 232–234°C.

Beispiel 15

1-(2-Cyano-6-n-hexanoylamino-phenoxy)-3-tert.butylamino-2-propanol-oxalat

Aus 10 g (0,03 Mol) Chlorhydrin und 12,6 ml tert. Butylamin werden nach Zugabe von Oxalsäure zum Aminoalkohol 5,6 g Oxalat erhalten.
Fp: 143–144°C.

Beispiel 16

1-(2-Cyano-4-n-hexanoylamino-phenoxy)-3-sec.butylamino-2-propanol

10 g (0,035 Mol) 1-(2-Cyano-4-hexanoylamino-phenoxy)-2,3-epoxypropan werden nach Lösen in 100 ml Äthanol und Zugabe von 7,3 g (0,1 Mol) sec. Butylamin eine Stunde zum Sieden unter Rückfluss erhitzt. Nach Abdestillieren des Lösungsmittels i.V. wird der Rückstand in der im Beispiel 6 beschriebenen Weise durch Säulenchromatographie gereinigt. Die so gewonnene Base wird in Essigester gelöst, mit verd. NaOH und Wasser gewaschen, getrocknet und zur Trockne eingeengt. Die verbleibende Testsubstanz wird aus Essigester unter Zugabe von Petroläther umkristallisiert. Eine anschliessende Umkristallisation aus Acetonitril ergibt 2,4 g reinweisse, kristalline Base.

Fp: 72–74°C.

In entsprechender Weise wurden die in den folgenden Beispielen 17 bis 19 genannten Verbindungen hergestellt:

Beispiel 17

1-(2-Cyano-4-n-octanoylamino-phenoxy)-3-sec.butylamino-2-propanol

Aus 10 g (0,0314 Mol) Epoxid und 7,3 g (0,1 Mol) sec. Butylamin wurden 3,7 g Base erhalten.

Fp: 83–86°C.

Beispiel 18

1-[2-Cyano-4-(3,3-dimethylbutyroyl)-amino-phenoxy]-3-sec.butylamino-2-propanol

Aus 14 g (0,0485 Mol) Epoxid und 11,7 g (0,16 Mol) sec. Butylamin erhielt man 6,0 g Base.

Beispiel 19

1-(2-Cyano-4-n-pentanoylamino-phenoxy)-3-tert.butylamino-2-propanol

10 g Epoxid und 6 g tert. Butylamin ergaben 5,8 g reines Amin.

Fp: 108–109°C.

Beispiel 20

1-(2-Cyano-4-isobutyroylamino-phenoxy)-3-isopropylamino-2-propanol

7,8 g (0,03 Mol) 1-(2-Cyano-4-isobutyroylamino-phenoxy)-2,3-epoxipropan werden nach Lösen in 100 ml Äthanol mit 10 ml Isopropylamin eine Stunde unter Rückfluss zum Sieden erhitzt. Nach Abdestillieren des Lösungsmittels wird der verbleibende Rückstand mit HCl angesäuert, mit Äther gewaschen, die wässrige Phase mit NaOH alkalisch gestellt und die ausfallende Base mit Essigester extrahiert. Der organische Extrakt wird mit $H_2O$ neutral gewaschen, über $Na_2SO_4$ getrocknet und i.V. zur Trockne eingeengt. Der Rückstand wird zweimal unter Verwendung von Aktivkohle umkristallisiert. Es werden 3,4 g reinweisse kristalline Base isoliert.

Fp: 126–127°C.

In gleicher Weise wurden die in den nachstehenden Beispielen 21 bis 24 genannten Verbindungen synthetisiert.

Beispiel 21

1-(2-Cyano-4-isobutyroylamino-phenoxy)-3-n-butylamino-2-propanol

Aus 7,8 g (0,03 Mol) Epoxid und 10 ml n-Butylamin wurden 2,8 g Base gewonnen.

Fp: 130–140°C.

Beispiel 22

1-(2-Cyano-4-isobutyroylamino-phenoxy)-3-sec.butylamino-2-propanol

Aus 7,8 g (0,03 Mol) Epoxid und 10 ml sec. Butylamin wurden 31 g Base erhalten.

Fp: 107–108°C.

Beispiel 23

1-(2-Cyano-4-n-octanoylamino-phenoxy)-3-isopropylamino-2-propanol

10 g (0,0314 Mol) Epoxid wurden mit 6,5 g (0,11 Mol) Isoorioylamin umgesetzt, wobei 4,0 g reine Base isoliert wurden.

Fp: 116–118°C.

Beispiel 24

1-(2-Cyano-4-isobutyroylamino-phenoxy)-3-tert.butylamino-2-propanol

Durch Umsetzung von 10 g Epoxid mit 10 ml tert. Butylamin wurden 3,7 g reine Base erhalten.

Fp: 118–121°C.

Beispiel 25

1-[2-Cyano-4-(2-äthylbutyroyl)-amino-phenoxy]-3-tert.butylamino-2-propanol-hydrochlorid

14 g (0,0485 Mol) 1-[2-Cyano-4-(2-äthylbutyroyl)-aminophenoxy]-2,3-epoxipropan werden in 100 ml Äthanol gelöst und nach Zugabe von 14,6 g (0,2 Mol) tert. Butylamin eine Stunde unter Rückfluss zum Sieden erhitzt. Nach Abdestillieren des Lösungsmittels i.V. wird der Rückstand mit HCl angesäuert, mit Äther ausgeschüttelt, die wässrige Phase über Kohle filtriert und mit $NH_4OH$ alkalisch gestellt. Die ölig ausfallende Base wird in Essigester aufgenommen, mit Wasser gewaschen und über $MgSO_4$ getrocknet. Nach Filtration und Abdestillieren des Lösungsmittels wird der Rückstand aus wenig Acetonitril umkristallisiert. Die reinweisse, kristalline Base wird in Acetonitril gelöst, alkoholische HCl zugegeben und das ausfallende Kristallisat abgesaugt. Es wird aus Äthanol unter Zugabe von Äther nochmals umkristallisiert. Man erhält 4,8 g Reinsubstanz vom Schmelzpunkt 214–217°C.

In Analogie zu Beispiel 25 wurden die in den nachstehenden Beispielen 26 bis 31 genannten Verbindungen synthetisiert.

Beispiel 26

1-[2-Cyano-4-(2-äthylbutyroyl)-amino-phenoxy]-3-sec. butylamino-2-propanol-hydrochlorid

Durch Reaktion von 14 g (0,0485 Mol) Epoxid mit 11,7 g (0,16 Mol) sec. Butylamin und Zugabe von HCl wurden 4,9 g Hydrochlorid gewonnen.

Fp: 185–188°C.

Beispiel 27

1-[2-Cyano-4-(2-äthylbutyroyl)-amino-phenoxy]-3-isopropylamino-2-propanol-hydrochlorid

Aus 14 g (0,0485 Mol) Epoxid und 11,8 g (0,2 Mol) Isopropylamin und späterer Zugabe von HCl wurden 5,2 g Hydrochlorid erhalten.
Fp: 216–218°C.

Beispiel 28
1-(2-Cyano-4-(3,3-dimethylbutyroyl)-amino-phenoxy]-3-isopropylamino-2-propanol-hydrochlorid
Durch Umsetzung von 14 g (0,0485 Mol) Epoxid und 11,8 g (0,2 Mol) Isopropylamin und anschliessende Zugabe von HCl wurden 6,5 g Hydrochlorid gewonnen.
Fp: 135–137°C.

Beispiel 29
1-[2-Cyano-4-(3,3-dimethylbutyroyl)-amino-phenoxy]-3-tert.butylamino-2-propanol-hydrochlorid
Nach Reaktion von 14 g (0,0485 Mol) Epoxid mit 11,6 g (0,16 Mol) tert. Butylamin und folgende Zugabe von HCl wurden 5,3 g Hydrochlorid erhalten.
Fp: 207–210°C.

Beispiel 30
1-(2-Cyano-4-n-butyroylamino-phenoxy)-3-tert.pentylamino-2-propanol-hydrochlorid
Aus 18 g Epoxid und 15 ml tert. Pentylamin entstanden bei nachfolgender Zugabe von HCl 7,6 g Hydrochlorid.
Fp: 144–146°C.

Beispiel 31
1-(2-Cyano-4-acetylamino-phenoxy)-3-isopropylamino-2-propanol-hydrochlorid
Die Umsetzung von 11 g Epoxid mit 10 g Isopropylamin ergab bei nachfolgendem Zusatz von HCl 3,8 g reines Hydrochlorid vom Schmelzpunkt 137–138°C.

B. Formulierungsbeispiele

1. Tabletten

| | |
|---|---|
| 1-(2-Cyano-4-n-hexanoylamino-phenoxy)-3-tert.-butylamino-2-propanol · HCl | 40,0 mg |
| Maisstärke | 164,0 mg |
| sek. Calciumphosphat | 240,0 mg |
| Magnesiumstearat | 1,0 mg |
| | 445,0 mg |

Herstellung: Die einzelnen Bestandteile werden intensiv miteinander vermischt und die Mischung in üblicher Weise granuliert. Das Granulat wird zu Tabletten von 445 mg Gewicht verpresst, von denen jede 40 mg Wirkstoff enthält.

Anstelle des in diesem Beispiel genannten Wirkstoffs können auch die Substanzen 1-(2-Cyano-6-isobutyroylamino-phenoxy)-3-(isopropyl)amino-2-propanol · oxalat und 1-(2-Cyano-6-(n-hexanoyl)amino-phenoxy)-3-(tert.butylamino)-2-propanol · oxalat in gleicher Menge verwendet werden.

2. Gelatine-Kapseln
Der Inhalt der Kapsel setzt sich wie folgt zusammen:

| | |
|---|---|
| 1-(2-Cyano-4-n-hexanoylamino-phenoxy)-3-(tert.butylamino)-2-propanol · HCl | 25,0 mg |
| Maisstärke | 175,0 mg |
| | 200,0 mg |

Herstellung: Die Bestandteile des Kapselinhalts werden intensiv vermischt und 200 mg-Portionen der Mischung werden in Gelatine-Kapseln geeigneter Grösse abgefüllt. Jede Kapsel enthält 25 mg des Wirkstoffs.

3. Injektionslösung
Die Lösung wird aus folgenden Bestandteilen hergestellt:

| | | |
|---|---|---|
| 1-(2-Cyano-4-acetylamino-phenoxy)-3-tert. butylamino-2-propanol | | 2,5 Teile |
| Natriumsalz der EDTA (Äthylendiamintetraessigsäure) | | 0,2 Teile |
| dest. Wasser | ad | 100,0 Teile |

Herstellung: Der Wirkstoff und das EDTA-Salz werden in genügend Wasser gelöst und mit Wasser auf das gewünschte Volumen aufgefüllt. Die Lösung wird frei von suspendierten Partikeln filtriert und in 1-ccm-Ampullen unter aseptischen Bedingungen abgefüllt. Zuletzt werden die Ampullen sterilisiert und verschlossen. Jede Ampulle enthält 25 mg Wirkstoff.

4. Depotdragées
Kern:

| | |
|---|---|
| (–)-1-(2-Cyano-4-n-hexanoylamino-phenoxy)-3-tert. butylamino-2-propanol · HCl | 25,0 g |
| Carboxymethylcellulose (CMC) | 295,0 g |
| Stearinsäure | 20,0 g |
| Celluloseacetatphthalat (CAP) | 40,0 g |
| | 380,0 g |

Herstellung: Der Wirkstoff, die CMC und die Stearinsäure werden intensiv gemischt und die Mischung in üblicher Weise granuliert, wobei man eine Lösung des CAP in 200 ml eines Gemisches aus Äthanol/Äthylacetat verwendet. Das Granulat wird dann zu 380-mg-Kernen verpresst, die in üblicher Weise mit einer zuckerhaltigen 5%igen Lösung von Polyvinylpyrrolidon in Wasser überzogen werden. Jedes Dragée enthält 25 mg Wirkstoff.

5. Tabletten

| | |
|---|---|
| 1-(2-Cyano-4-n-hexanoylamino-phenoxy)-3-isopropylamin-2-propanol | 35,0 g |
| 2,6-Bis-(diäthanolamino)-4,8-dipiperidino-pyrimido-[5,4-d]-pyrimidin | 75,0 g |
| Milchzucker | 164,0 g |
| Maisstärke | 194,0 g |
| kolloidale Kieselsäure | 14,0 g |

| | |
|---|---|
| Polyvinylpyrrolidon | 6,0 g |
| Magnesiumstearat | 2,0 g |
| lösliche Stärke | 10,0 g |
| | 500,0 g |

Anstelle des in diesem Beispiel genannten β-adrenolytisch wirksamen Stoffs kann auch die Substanz 1-(2-Cyano-6-isobutyroylamino-phenoxy)-3-(isopropylamino)-2-propanol · oxalat in gleicher Menge verwendet werden.

Herstellung: Der Wirkstoff wird zusammen mit dem Milchzucker, der Maisstärke, der kolloidalen Kieselsäure und dem Polyvinylpyrrolidon nach intensiver Durchmischung in üblicher Weise granuliert, wobei man eine wässrige Lösung der löslichen Stärke verwendet. Das Granulat wird mit dem Magnesiumstearat gemischt und zu 1000 Tabletten von je 500 mg Gewicht verpresst, die je 35 mg des ersten und 75 mg des zweiten Wirkstoffs enthalten.

**Patentansprüche**

für die Vertragsstaaten BE, CH, DE, FR, LI, IT, LU, NL, SE

1. Verbindungen der allgemeinen Formel

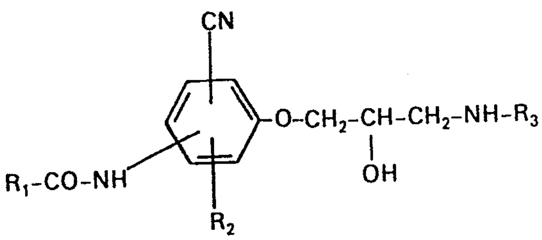

I

in der

$R_1$ einen geraden oder verzweigten Alkylrest mit 1 bis 20 C-Atomen,

$R_2$ ein Wasserstoff- oder Halogenatom, eine gerade oder verzweigte Alkyl- oder Alkoxygruppe mit 1 bis 4 C-Atomen oder die zweiwertigen Gruppen $-CH=CH-CH=CH-$ oder $-(CH_2)_n-$ (n = ganze Zahl von 3 bis 5) mit Bindung der freien Valenzen in o-Stellung zueinander,

$R_3$ einen geraden oder verzweigten Alkylrest mit 3 bis 10 C-Atomen, wobei $R_3$ von Isopropyl und von tert. Butyl verschieden ist, wenn $R_1$ Äthyl oder n-Propyl und gleichzeitig $R_2$ Wasserstoff bedeutet

sowie ihre Säureadditionssalze.

2. Verbindungen der allgemeinen Formel I, dadurch gekennzeichnet, dass $R_1$ gerades oder verzweigtes Alkyl mit 1 bis 7 C-Atomen, $R_2$ Wasserstoff und $R_3$ einen in der Nachbarschaft zur Aminogruppe verzweigten Alkylrest bedeuten, sowie deren physiologisch verträgliche Säureadditionssalze.

3. 1-(2-Cyano-4-n-hexanoylamino-phenoxy)-3-(tert.butylamino)-2-propanol bzw. seine Salze.

4. 1-(2-Cyano-4-n-hexanoylamino-phenoxy)-3-isopropylamino-2-propanol bzw. seine Salze.

5. Pharmazeutische Zubereitungen, enthaltend Substanzen der allgemeinen Formel I bzw. ihre physiologisch verträglichen Säureadditionssalze in Kombination mit üblichen Hilfs- und/oder Trägerstoffen.

6. Pharmazeutische Präparate, enthaltend Substanzen der allgemeinen Formel I bzw. ihre physiologisch verträglichen Säureadditionssalze in Kombination mit anderen pharmazeutischen Wirkstoffen sowie üblichen Hilfs- und/oder Trägerstoffen.

7. Substanzen der allgemeinen Formel I bzw. ihre physiologisch verträglichen Säureadditionssalze zur Behandlung der Tachycardie.

8. Substanzen der allgemeinen Formel I bzw. ihre physiologisch verträglichen Säureadditionssalze zur Behandlung von Hypertonie.

9. Substanzen der allgemeinen Formel I bzw. ihre physiologisch verträglichen Säureadditionssalze zur Behandlung und Prophylaxe von Erkrankungen der Herzkranzgefässe.

10. Verfahren zur Herstellung von pharmazeutischen Präparaten nach Anspruch 5, dadurch gekennzeichnet, dass man Verbindungen der Formel I bzw. ihre physiologisch verträglichen Säureadditionssalze mit üblichen galenischen Hilfs- und/oder Trägerstoffen in üblichen pharmazeutischen Anwendungsformen verarbeitet.

11. Verfahren zur Herstellung von pharmazeutischen Zubereitungen nach Anspruch 6, dadurch gekennzeichnet, dass man Substanzen der allgemeinen Formel I in Kombination mit anderen Wirkstoffen sowie üblichen galenischen Hilfs- und/oder Trägerstoffen zu üblichen pharmazeutischen Anwendungsformen verarbeitet.

12. Verfahren zur Herstellung von Verbindungen der allgemeinen Formel I

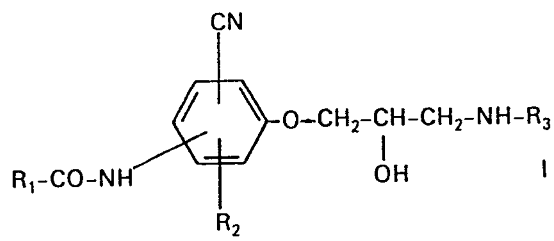

I

worin $R_1$ bis $R_3$ die oben genannte Bedeutung haben, dadurch gekennzeichnet, dass man

a) eine Verbindung der allgemeinen Formel II

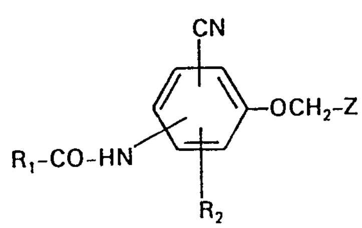

II

in der $R_1$ und $R_2$ wie in Formel I definiert sind und Z die Gruppe

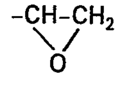

oder $-CHOH-CH_2-Hal$ (Hal = Halogen) bedeutet, mit einem Amin der allgemeinen Formel

$NH_2R_3$ III

in der $R_3$ wie in Formel I definiert ist, umsetzt, oder dass man

b) ein Oxazolidin-Derivat der allgemeinen Formel IV

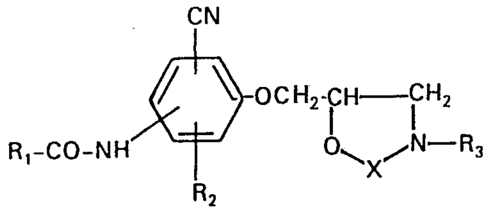

IV

in der $R_1$ bis $R_3$ wie in Formel I und X eine $-CO-$, $-CH_2-$ oder CH-Niederalkylgruppe bedeutet, definiert sind, hydrolysiert, oder dass man

c) eine Verbindung der allgemeinen Formel V

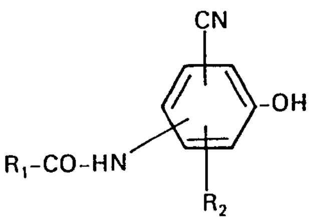

V

in welcher $R_1$ und $R_2$ wie in Formel I definiert sind (oder ein Salz dieser Verbindung) mit einem Azetidinolderivat der allgemeinen Formel

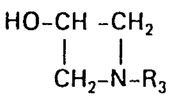

VI

in welcher $R_3$ wie in Formel I definiert ist, in wasserfreiem Medium umsetzt,

und die nach einem der Verfahren a) bis c) erhaltenen Verbindungen gewünschtenfalls in ihre Säureadditionssalze überführt.

13. Verfahren zur Herstellung von optisch aktiven Verbindungen der allgemeinen Formel I, dadurch gekennzeichnet, dass man

a) von optisch aktivem Ausgangsmaterial ausgeht, oder dass man

b) die nach einem der Verfahren des Anspruchs 12 erhaltenen Verbindungen durch Umsetzung mit optisch aktiven Hilfssäuren in ihre diastereomeren Salze überführt und letztere durch fraktionierte Kristallisation auftrennt.

**Patentansprüche**

für den Vertragsstaat AT

1. Verfahren zur Herstellung von Verbindungen der allgemeinen Formel I

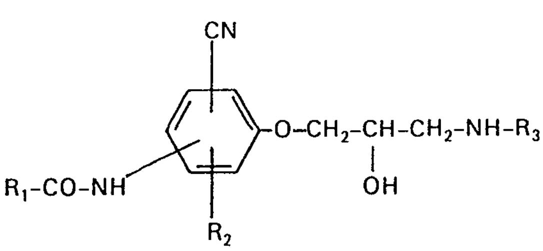

I

worin

$R_1$ einen geraden oder verzweigten Alkylrest mit 1 bis 20 C-Atomen,

$R_2$ ein Wasserstoff- oder Halogenatom, eine gerade oder verzweigte Alkyl- oder Alkoxygruppe mit 1 bis 4 C-Atomen oder die zweiwertigen Gruppen $-CH=CH-CH=CH-$ oder $-(CH_2)_n-$ (n = ganze Zahl von 3 bis 5) mit Bindung der freien Valenzen in o-Stellung zueinander,

$R_3$ einen geraden oder verzweigten Alkylrest mit 3 bis 10 C-Atomen, wobei $R_3$ von Isopropyl und von tert. Butyl verschieden ist, wenn $R_1$ Äthyl oder n-Propyl und gleichzeitig $R_2$ Wasserstoff bedeutet

bedeuten, dadurch gekennzeichnet, dass man

a) eine Verbindung der allgemeinen Formel II

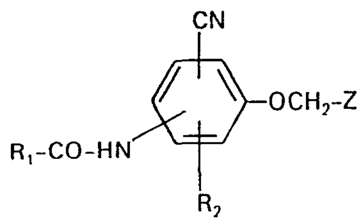

II

in der $R_1$ und $R_2$ wie in Formel I definiert sind und Z die Gruppe

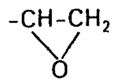

oder $-CHOH-CH_2-Hal$ (Hal = Halogen) bedeutet, mit einem Amin der allgemeinen Formel

$NH_2-R_3$ III

in der $R_3$ wie in Formel I definiert ist, umsetzt, oder dass man

b) ein Oxazolidin-Derivat der allgemeinen Formel IV

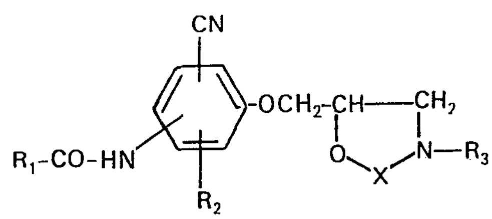

IV

in der $R_1$ bis $R_3$ wie in Formel I und X eine $-CO-$, $-CH_2-$ oder $-CH$-Niederalkylgruppe bedeutet, definiert sind, hydrolysiert, oder dass man

c) eine Verbindung der allgemeinen Formel V

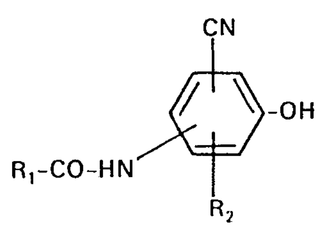

V

in welcher $R_1$ und $R_2$ wie in Formel I definiert sind (oder ein Salz dieser Verbindung) mit einem Azetidinolderivat der allgemeinen Formel VI

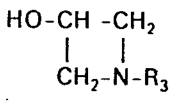

VI

in welcher $R_3$ wie in Formel I definiert ist, in wasserfreiem Medium umsetzt,
und die nach einem der Verfahren a) bis c) erhaltenen Verbindungen gewünschtenfalls in ihre Säureadditionssalze überführt.

2. Verfahren zur Herstellung von optisch aktiven Verbindungen der allgemeinen Formel I, dadurch gekennzeichnet, dass man
a) von optisch aktivem Ausgangsmaterial ausgeht, oder dass man
b) die nach einem Verfahren des Anspruchs 1 erhaltenen Verbindungen durch Umsetzung mit optisch aktiven Hilfssäuren in ihre diastereomeren Salze überführt und letztere durch fraktionierte Kristallisation auftrennt.

**Claims**

(for the contracting states BE, CH, DE, FR, LI, IT, LU, NL, SE)

1. Compounds of general formula

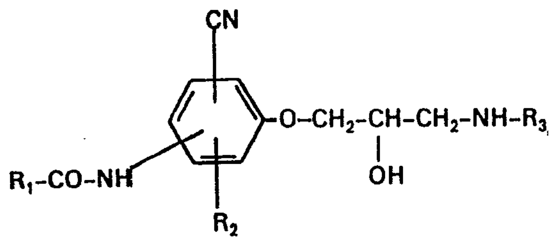

I

wherein
$R_1$ represents a straight-chained or branched alkyl group with 1 to 20 carbon atoms,
$R_2$ represents a hydrogen or halogen atom, a straight-chained or branched alkyl or alkoxy group with 1 to 4 carbon atoms or the divalent groups $-CH=CH-CH=CH$ or $(CH_2)_n$ ($n$ = an integer from 3 to 5): with the free valencies bonded in the o-position relative to one another,
$R_3$ represents a straight-chained or branched alkyl group with 3 to 10 carbon atoms, wherein $R_3$ is different from isopropyl and tert.butyl if $R_1$ represents ethyl or n-propyl and at the same time $R_2$ represents hydrogen,
and the acid addition salts thereof.

2. Compounds of general formula I, characterised in that $R_1$ represents straight-chained or branhced alkyl with 1 to 7 carbon atoms, $R_2$ represents hydrogen and $R_3$ represents an alkyl group which is branched in the vicinity of the amino group, and the physiologically acceptable acid addition salts thereof.

3. 1-(2-Cyano-4-n-hexanoylamino)-phenoxy-3-(tert.butylamino)-2-propanol and the salts thereof.

4. 1-(2-Cyano-4-n-hexanoylamino-phenoxy)-3-isopropylamino-2-propanol and the salts thereof.

5. Pharmaceutical preparations containing substances of general formula I or the physiologically acceptable acid addition salts thereof combined with conventional excipients and/or carriers.

6. Pharmaceutical preparations containing substances of general formula I or the physiologically acceptable acid addition salts thereof combined with other pharmaceutically active ingredients and conventional excipients and/or carriers.

7. Substances of general formula I or the physiologically acceptable acid addition salts thereof for the treatment of tachycardia.

8. Substances of general formula I or the physiologically acceptable acid addition salts thereof for the treatment of hypertonia.

9. Substances of general formula I or the physiologically acceptable acid addition salts thereof for the treatment and prophylaxis of diseases of the coronary vessels.

10. Process for preparing pharmaceutical preparations according to claim 5, characterised in that compounds of formula I or the physiologically acceptable acid addition salts thereof are formulated with conventional galenic excipients and/or carriers to form conventional pharmaceutical forms.

11. Process for preparing pharmaceutical preparations according to claim 6, characterised in that substances of general formula I combined with other active substances and conventional galenic excipients and/or carriers are processed to form conventional pharmaceutical forms of administration.

12. Process for preparing compounds of general formula I

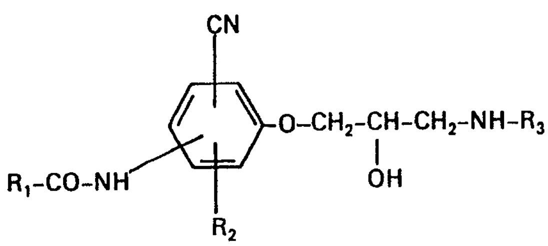

I

wherein $R_1$ to $R_3$ are as hereinbefore defined, characterised in that
a) a compound of general formula II

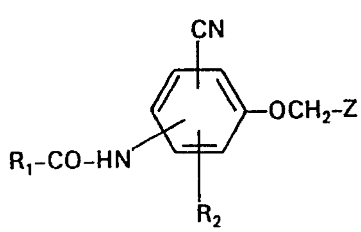

II

wherein $R_1$ and $R_2$ are as defined in formula I and Z represents the group

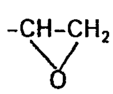

or $-CHOH-CH_2-Hal$ (Hal = halogen), is reacted with an amine of general formula

$NH_2-R_3$ III

wherein $R_3$ is defined as in formula I, or in that

b) an oxazolidine derivative of general formula IV

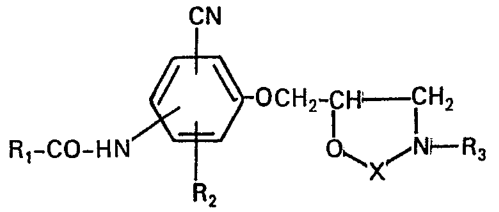

IV

wherein $R_1$ to $R_3$ are as defined in formula I and X represents a -CO-, $-CH_2$ or -CH- lower alkyl group, is hydrolysed, or in that

c) a compound of general formula V

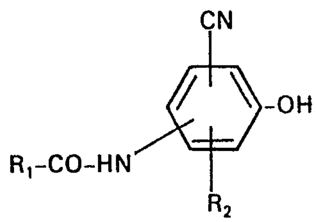

V

wherein $R_1$ and $R_2$ are as defined in formula I (or a salt of this compound) is reacted with an azetidinol derivative of general formula VI

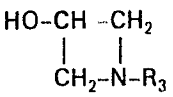

VI

wherein $R_3$ is as defined in formula I, in an anhydrous medium, and the compounds obtained according to one of the processes a) to c) are converted, if desired, into the acid addition salts thereof.

13. Process for preparing optically active compounds of geneal formula I, characterised in that

a) optically active starting materials are used, or in that

b) the compounds obtained according to one of the processes in claim 12 are converted into their diastereomeric salts by reacting with optically active auxiliary acids and these salts are separated by fractional crystallisation.

**Revendications**

(pour les Etats contractants BE, CH, DE, FR, LI, IT, LU, NL, SE)

1. Composés de formule générale:

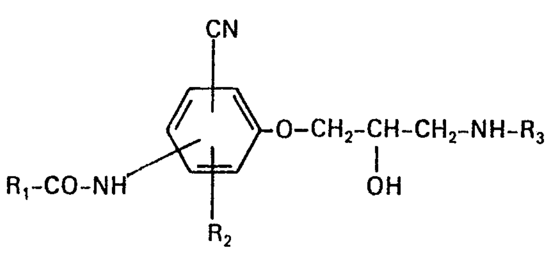

I

dans laquelle:

$R_1$ représente un radical alcoyle rectiligne ou ramifié avec 1 à 20 atomes de C,

$R_2$ représente un atome d'hydrogène ou d'halogène, un groupe alcoyle ou alcoxy rectiligne ou ramifié avec 1 à 4 atomes de C ou les groupes bivalents $-CH=CH-CH=CH-$ ou $-(CH_2)_n-$ (n = nombre entier de 3 à 5) avec liaison des valences libres en position o l'une par rapport à l'autre,

$R_3$ représente un radical alcoyle rectiligne ou ramifié avec 3 à 10 atomes de C, $R_3$ étant différent d'isopropyle et de tert.butyle lorsque $R_1$ représente éthyle ou n-propyle et en même temps $R_2$ représente l'hydrogène,

ainsi que leurs sels d'addition d'acide.

2. Composés de formule générale I, caractérisés en ce que $R_1$ représente un alcoyle rectiligne ou ramifié avec 1 à 7 atomes de C, $R_2$ représente l'hydrogène et $R_3$ représente un radical alcoyle ramifié dans le voisinage du groupe amino, ainsi que leurs sels d'addition d'acide physiologiquement supportables.

3. Le 1-(2-cyano-4-n-hexanoylamino)-phénoxy-3-(tert.butylamino)-2-propanol ou respectivement ses sels.

4. Le 1-(2-cyano-4-n-hexanoylamino-phénoxy)-3-isopropylamino-2-propanopl ou respectivement ses sels.

5. Préparations pharmaceutiques contenant des substances de formule générale I ou respectivement leurs sels d'addition d'acide physiologiquement supportables en combinaison avec des adjuvants et/ou excipients habituels.

6. Préparations pharmaceutiques contenant des substances de formule générale I ou respectivement leurs sels d'addition d'acide physiologiquement supportables en combinaison avec d'autres substances actives pharmaceutiques ainsi que des adjuvants et/ou excipients habituels.

7. Substances de formule générale I ou respectivement leurs sels d'addition d'acide physiologiquement supportables, pour le traitement de la tachycardie.

8. Substances de formule générale I ou respectivement leurs sels d'addition d'acide physiologiquement supportables, pour le traitement de l'hypertonie.

9. Substances de formule générale I ou respectivement leurs sels d'addition d'acide physiologiquement supportables, pour le traitement et la prophylaxie des maladies des vaisseaux coronaires du cœur.

10. Procédé de fabrication de préparations pharmaceutiques selon la revendication 5, caractérisé en ce que l'on transforme des composés de formule I ou respectivement leurs sels d'addition d'acide physiologiquement supportables avec des adjuvants et/ou excipients galéniques habituels en formes d'utilisation pharmaceutiques habituelles.

11. Procédé de fabrication de préparations pharmaceutiques selon la revendication 6, caractérisé en ce que l'on transforme des substances de formule générale I en combinaison avec d'autres substances actives ainsi qu'avec des adjuvants et/ou excipients galéniques habituels en formes d'utilisation pharmaceutiques habituelles.

12. Procédé de préparation des composés de formule générale I:

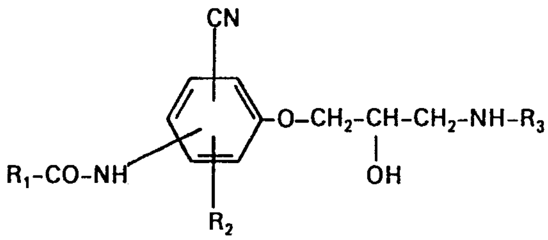

I

dans laquelle $R_1$ à $R_3$ ont la signification indiquée plus haut, caractérisé en ce que:

a) on fait réagir un composé de formule générale II:

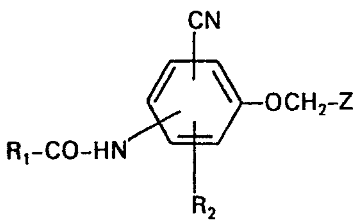

II

dans laquelle $R_1$ et $R_2$ sont définis comme dans la formule I et Z représente le groupe

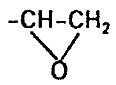

ou $-CHOH-CH_2-hal$ (hal = halogène), avec une amine de formule générale:

$NH_2R_3$ III

dans laquelle $R_3$ est défini comme dans la formule I, ou en ce que:

b) on hydrolyse un dérivé d'oxazolidine de formule générale IV:

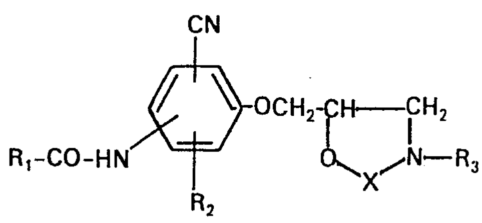

IV

dans laquelle $R_1$ à $R_3$ sont définis comme dans la formule I et X représente un groupe $-CO-$, $-CH_2-$ ou CH-alcoyle inférieur, ou en ce que:

c) on fait réagir un composé de formule générale V:

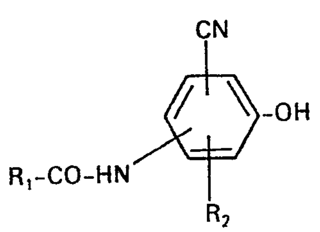

V

dans laquelle $R_1$ et $R_2$ sont définis comme dans la formule I (ou un sel de ce composé) avec un dérivé d'azétidinol de formule générale:

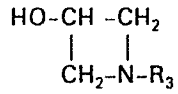

VI

dans laquelle $R_3$ est défini comme dans la formule I, en milieu anhydre, et on transforme si on le désire les composés obtenus selon l'un des procédés a) à c) en leurs sels d'addition d'acide.

13. Procédé de préparation de composés optiquement actifs de formule générale I, caractérisé en ce que:

a) on part de matériaux de départ optiquement actifs, ou en ce que:

b) on transforme les composés obtenus selon l'un des procédés de la revendication 12 par réaction avec des acides adjuvants optiquement actifs en leurs sels diastéréoisomères et on sépare ces derniers par cristallisation fractionnée.

**Claims**

for the contracting state AT

1. Process for preparing compounds of general formula I

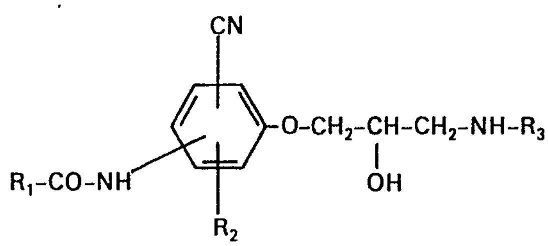

I

wherein

$R_1$ represents a straight-chained or branched alkyl group with 1 to 20 carbon atoms,

$R_2$ represents a hydrogen or halogen atom, a straight-chained or branched alkyl or alkoxy group with 1 to 4 carbon atoms or the divalent groups $-CH=CH-CH=CH$ or $(CH_2)_n$ (n = an integer from 3 to 5) with the free valencies bonded in the o-positon relative to one another,

$R_3$ represents a straight-chained or branched alkyl group with 3 to 10 carbon atoms, wherein $R_3$ is different from isopropyl and tert.butyl if $R_1$ represents ethyl or n-propyl and at the same time $R_2$ represents hydrogen, characterised in that

a) a compound of general formula II

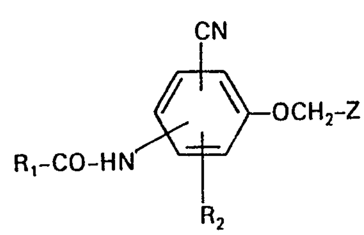

II

wherein $R_1$ and $R_2$ are as defined in formula I and Z represents the group

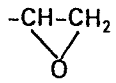

or $-CHOH-CH_2-Hal$ (Hal = halogen), is reacted with an amine of general formula

$NH_2-R_3$ III

wherein $R_3$ is defined as in formula I, or in that
b) an oxazolidine derivative of general formula IV

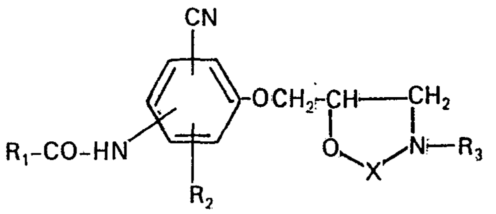

IV

wherein $R_1$ to $R_3$ are as defined in formula I and X represents a -CO-, $-CH_2-$ or -CH- lower alkyl group, is hydrolysed, or in that
c) a compound of general formula V

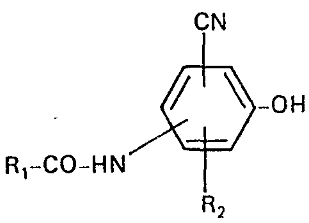

V

wherein $R_1$ and $R_2$ are as defined in formula I (or a salt of this compound) is reacted with an azetidinol derivative of general formula VI

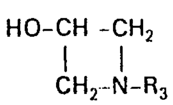

VI

wherein $R_3$ is as defined in formula I, and the compounds obtained according to one of the processes a) to c) are converted, if desired, into the acid addition salts thereof.

2. Process for preparing optically active compounds of general formula I, characterised in that
a) optically active starting materials are used, or in that
b) the compounds obtained according to one of the processes in claim 1 are converted into their diastereomeric salts by reacting with optically active auxiliary acids and these salts are separated by fractional crystallisation.

**Revendications**

(pour l'Etat contractant AT).

1. Procécé de préparation de composés de formule générale I:

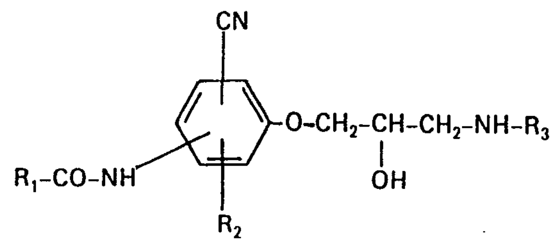

I

dans laquelle:
$R_1$ représente un radical alcoyle rectiligne ou ramifié avec 1 à 20 atomes de C,
$R_2$ représente un atome d'hydrogène ou d'halogène, un groupe alcoyle ou alcoxy rectiligne ou ramifié avec 1 à 4 atomes de C, ou les groupes bivalents -CH=CH-CH=CH- ou $-(CH_2)_n-$ (n = nombre entier de 3 à 5) avec liaison des valences libres en position o l'une par rapport à l'autre,
$R_3$ représente un radical alcoyle rectiligne ou ramifié avec 3 à 10 atomes de C, $R_3$ étant différent d'isopropyle et de tert.butyle lorsque $R_1$ représente éthyle ou n-propyle et en même temps $R_2$ représente l'hydrogène, caractérisé en ce que:
a) on fait réagir un composé de formule générale II:

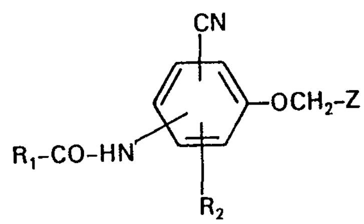

II

dans laquelle $R_1$ et $R_2$ sont définis comme dans la formule I et Z représente le groupe

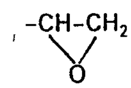

ou $-CHOH-CH_2-hal$ (hal = halogène), avec une amine de formule générale:

$NH_2-R_3$ III

dans laquelle $R_3$ est défini comme dans la formule I, ou en ce que:
b) on hydrolyse un dérivé d'oxazolidine de formule générale IV:

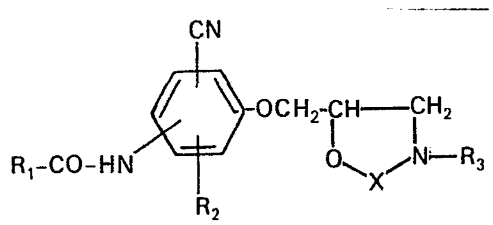

IV

dans laquelle $R_1$ à $R_3$ sont définis comme dans la formule I et X représente un groupe -CO-, $-CH_2-$ ou -CH-alcoyle inférieur, ou en ce que:

$NH_2-R_3$ III

wherein $R_3$ is defined as in formula I, or in that
b) an oxazolidine derivative of general formula IV

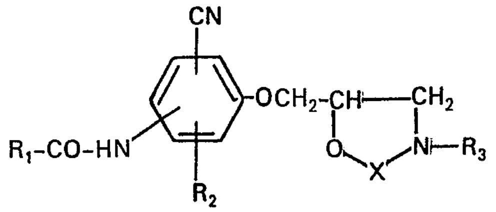
IV

wherein $R_1$ to $R_3$ are as defined in formula I and X represents a -CO-, $-CH_2$ or -CH- lower alkyl group, is hydrolysed, or in that
c) a compound of general formula V

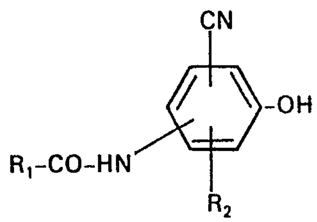
V

wherein $R_1$ and $R_2$ are as defined in formula I (or a salt of this compound) is reacted with an azetidinol derivative of general formula VI

$HO-CH-CH_2$
| |
$CH_2-N-R_3$ VI

wherein $R_3$ is as defined in formula I, in an anhydrous medium, and the compounds obtained according to one of the processes a) to c) are converted, if desired, into the acid addition salts thereof.

13. Process for preparing optically active compounds of geneal formula I, characterised in that
a) optically active starting materials are used, or in that
b) the compounds obtained according to one of the processes in claim 12 are converted into their diastereomeric salts by reacting with optically active auxiliary acids and these salts are separated by fractional crystallisation.

**Revendications**

(pour les Etats contractants BE, CH, DE, FR, LI, IT, LU, NL, SE)

1. Composés de formule générale:

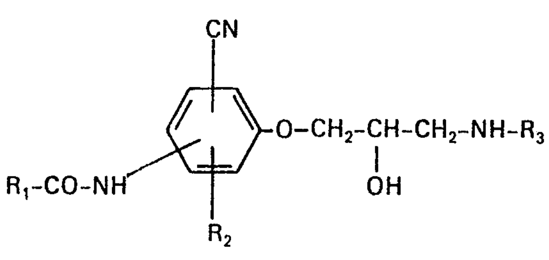
I

dans laquelle:
$R_1$ représente un radical alcoyle rectiligne ou ramifié avec 1 à 20 atomes de C,
$R_2$ représente un atome d'hydrogène ou d'halogène, un groupe alcoyle ou alcoxy rectiligne ou ramifié avec 1 à 4 atomes de C ou les groupes bivalents $-CH=CH-CH=CH-$ ou $-(CH_2)_n-$ (n = nombre entier de 3 à 5) avec liaison des valences libres en position o l'une par rapport à l'autre,
$R_3$ représente un radical alcoyle rectiligne ou ramifié avec 3 à 10 atomes de C, $R_3$ étant différent d'isopropyle et de tert.butyle lorsque $R_1$ représente éthyle ou n-propyle et en même temps $R_2$ représente l'hydrogène,
ainsi que leurs sels d'addition d'acide.

2. Composés de formule générale I, caractérisés en ce que $R_1$ représente un alcoyle rectiligne ou ramifié avec 1 à 7 atomes de C, $R_2$ représente l'hydrogène et $R_3$ représente un radical alcoyle ramifié dans le voisinage du groupe amino, ainsi que leurs sels d'addition d'acide physiologiquement supportables.

3. Le 1-(2-cyano-4-n-hexanoylamino)-phénoxy-3-(tert.butylamino)-2-propanol ou respectivement ses sels.

4. Le 1-(2-cyano-4-n-hexanoylamino-phénoxy)-3-isopropylamino-2-propanopl ou respectivement ses sels.

5. Préparations pharmaceutiques contenant des substances de formule générale I ou respectivement leurs sels d'addition d'acide physiologiquement supportables en combinaison avec des adjuvants et/ou excipients habituels.

6. Préparations pharmaceutiques contenant des substances de formule générale I ou respectivement leurs sels d'addition d'acide physiologiquement supportables en combinaison avec d'autres substances actives pharmaceutiques ainsi que des adjuvants et/ou excipients habituels.

7. Substances de formule générale I ou respectivement leurs sels d'addition d'acide physiologiquement supportables, pour le traitement de la tachycardie.

8. Substances de formule générale I ou respectivement leurs sels d'addition d'acide physiologiquement supportables, pour le traitement de l'hypertonie.

9. Substances de formule générale I ou respectivement leurs sels d'addition d'acide physiologiquement supportables, pour le traitement et la prophylaxie des maladies des vaisseaux coronaires du cœur.

10. Procédé de fabrication de préparations pharmaceutiques selon la revendication 5, caractérisé en ce que l'on transforme des composés de formule I ou respectivement leurs sels d'addition d'acide physiologiquement supportables avec des adjuvants et/ou excipients galéniques habituels en formes d'utilisation pharmaceutiques habituelles.

11. Procédé de fabrication de préparations pharmaceutiques selon la revendication 6, caractérisé en ce que l'on transforme des substances de formule générale I en combinaison avec d'autres substances actives ainsi qu'avec des adjuvants et/ou excipients galéniques habituels en formes d'utilisation pharmaceutiques habituelles.

12. Procédé de préparation des composés de formule générale I:

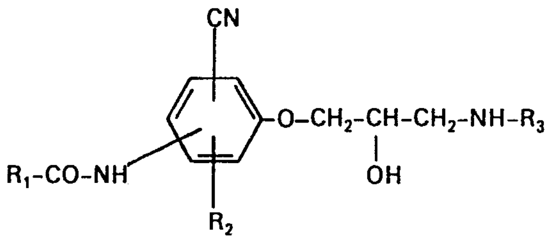

I

dans laquelle $R_1$ à $R_3$ ont la signification indiquée plus haut, caractérisé en ce que:

a) on fait réagir un composé de formule générale II:

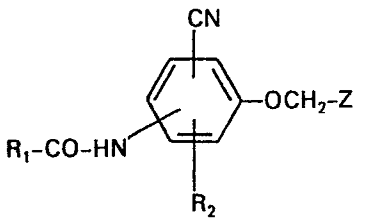

II

dans laquelle $R_1$ et $R_2$ sont définis comme dans la formule I et Z représente le groupe

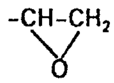

ou $-CHOH-CH_2-hal$ (hal = halogène), avec une amine de formule générale:

$NH_2R_3$ III

dans laquelle $R_3$ est défini comme dans la formule I, ou en ce que:

b) on hydrolyse un dérivé d'oxazolidine de formule générale IV:

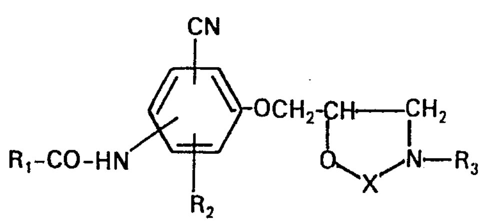

IV

dans laquelle $R_1$ à $R_3$ sont définis comme dans la formule I et X représente un groupe $-CO-$, $-CH_2-$ ou CH-alcoyle inférieur, ou en ce que:

c) on fait réagir un composé de formule générale V:

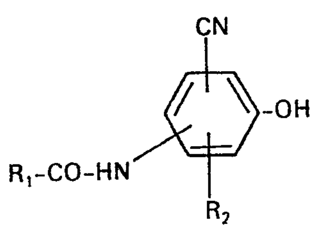

V

dans laquelle $R_1$ et $R_2$ sont définis comme dans la formule I (ou un sel de ce composé) avec un dérivé d'azétidinol de formule générale:

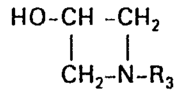

VI

dans laquelle $R_3$ est défini comme dans la formule I, en milieu anhydre, et on transforme si on le désire les composés obtenus selon l'un des procédés a) à c) en leurs sels d'addition d'acide.

13. Procédé de préparation de composés optiquement actifs de formule générale I, caractérisé en ce que:

a) on part de matériaux de départ optiquement actifs, ou en ce que:

b) on transforme les composés obtenus selon l'un des procédés de la revendication 12 par réaction avec des acides adjuvants optiquement actifs en leurs sels diastéréoisomères et on sépare ces derniers par cristallisation fractionnée.

**Claims**

for the contracting state AT

1. Process for preparing compounds of general formula I

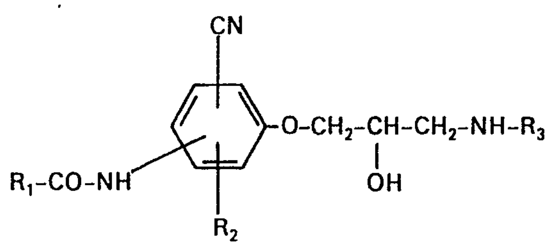

I

wherein

$R_1$ represents a straight-chained or branched alkyl group with 1 to 20 carbon atoms,

$R_2$ represents a hydrogen or halogen atom, a straight-chained or branched alkyl or alkoxy group with 1 to 4 carbon atoms or the divalent groups $-CH=CH-CH=CH$ or $(CH_2)_n$ (n = an integer from 3 to 5) with the free valencies bonded in the o-positon relative to one another,

$R_3$ represents a straight-chained or branched alkyl group with 3 to 10 carbon atoms, wherein $R_3$ is different from isopropyl and tert.butyl if $R_1$ represents ethyl or n-propyl and at the same time

$R_2$ represents hydrogen, characterised in that

a) a compound of general formula II

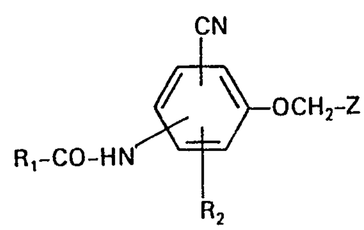

II

wherein $R_1$ and $R_2$ are as defined in formula I and Z represents the group

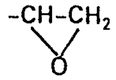

or $-CHOH-CH_2-Hal$ (Hal = halogen), is reacted with an amine of general formula

$NH_2-R_3$ III

wherein $R_3$ is defined as in formula I, or in that
b) an oxazolidine derivative of general formula IV

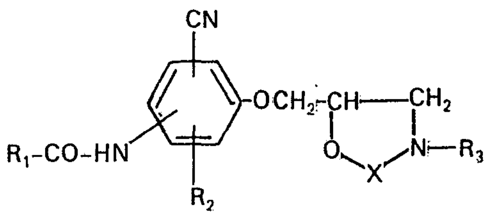
IV

wherein $R_1$ to $R_3$ are as defined in formula I and X represents a $-CO-$, $-CH_2-$ or $-CH-$ lower alkyl group, is hydrolysed, or in that
c) a compound of general formula V

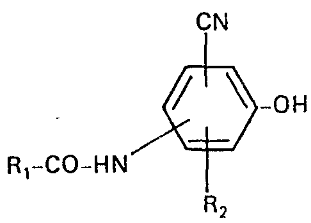
V

wherein $R_1$ and $R_2$ are as defined in formula I (or a salt of this compound) is reacted with an azetidinol derivative of general formula VI

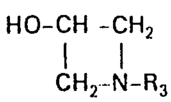
VI

wherein $R_3$ is as defined in formula I, and the compounds obtained according to one of the processes a) to c) are converted, if desired, into the acid addition salts thereof.

2. Process for preparing optically active compounds of general formula I, characterised in that
a) optically active starting materials are used, or in that
b) the compounds obtained according to one of the processes in claim 1 are converted into their diastereomeric salts by reacting with optically active auxiliary acids and these salts are separated by fractional crystallisation.

**Revendications**

(pour l'Etat contractant AT).

1. Procécé de préparation de composés de formule générale I:

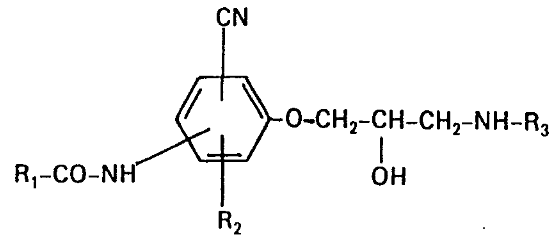
I

dans laquelle:
$R_1$ représente un radical alcoyle rectiligne ou ramifié avec 1 à 20 atomes de C,
$R_2$ représente un atome d'hydrogène ou d'halogène, un groupe alcoyle ou alcoxy rectiligne ou ramifié avec 1 à 4 atomes de C, ou les groupes bivalents $-CH=CH-CH=CH-$ ou $-(CH_2)_n-$ (n = nombre entier de 3 à 5) avec liaison des valences libres en position o l'une par rapport à l'autre,
$R_3$ représente un radical alcoyle rectiligne ou ramifié avec 3 à 10 atomes de C, $R_3$ étant différent d'isopropyle et de tert.butyle lorsque $R_1$ représente éthyle ou n-propyle et en même temps $R_2$ représente l'hydrogène, caractérisé en ce que:
a) on fait réagir un composé de formule générale II:

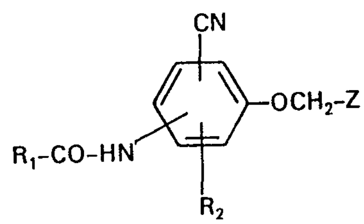
II

dans laquelle $R_1$ et $R_2$ sont définis comme dans la formule I et Z représente le groupe

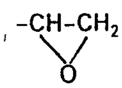

ou $-CHOH-CH_2-hal$ (hal = halogène), avec une amine de formule générale:

$NH_2-R_3$ III

dans laquelle $R_3$ est défini comme dans la formule I, ou en ce que:
b) on hydrolyse un dérivé d'oxazolidine de formule générale IV:

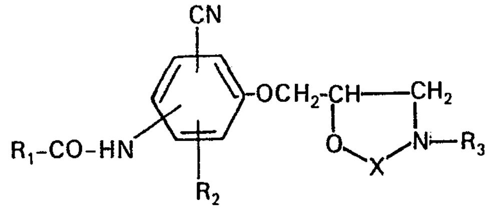
IV

dans laquelle $R_1$ à $R_3$ sont définis comme dans la formule I et X représente un groupe $-CO-$, $-CH_2-$ ou $-CH$-alcoyle inférieur, ou en ce que:

c) on fait réagir un composé de formule générale V:

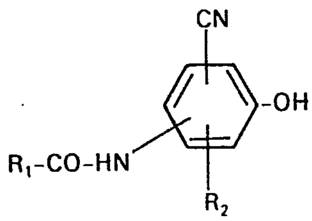

V

dans laquelle $R_1$ et $R_2$ sont définis comme dans la formule I (ou un sel de ce composé) avec un dérivé d'azétidinol de formule générale VI:

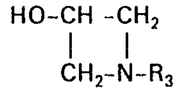

VI

dans laquelle $R_3$ est défini comme dans la formule I, en milieu anhydre, et en ce que l'on transforme si on le désire les composés obtenus selon l'un des procédés a) à c) en leurs sels d'addition d'acide.

2. Procédé de préparation de composés optiquement actifs de formule générale I, caractérisé en ce que:

a) on part de matière de départ optiquement active, ou en ce que:

b) on transforme les composés obtenus selon l'un des procédés de la revendication 1 par réaction avec des acides adjuvants optiquement actifs en leurs sels diastéréoisomères et on sépare ces derniers par cristallisation fractionnée.